# EUROPEAN PATENT SPECIFICATION

(11) **EP 3 432 177 B1**
(45) Date of publication and mention of the grant of the patent: **26.04.2023**
(21) Application number: 17181726.5
(22) Date of filing: 17.07.2017
(51) Int. Cl.: G16B 40/00, G16B 40/10

(54) **METHOD AND DEVICE FOR ANALYZING A DATASET**
VERFAHREN UND VORRICHTUNG ZUR ANALYSE EINES DATENSATZES
PROCÉDÉ ET SYSTÈME DE CHAUFFAGE D'UN FOUR

(43) Date of publication of application: 23.01.2019
(73) Proprietor: Roche Diagnostics GmbH, 68305 Mannheim (DE); F. Hoffmann-La Roche AG, 4070 Basel (CH)
(72) Inventor: Knobel, Rolf, 6343 Rotkreuz (CH)
(74) Representative: Altmann Stößel Dick Patentanwälte PartG mbB

(56) References cited:
- WO-A1-99/54724

## Description

The present invention is concerned with data evaluation tools. In particular, it relates to a computer-implemented method for analyzing a dataset, the method comprising a step of performing an analyzing assay and retrieving intensity values from said assay, wherein said analyzing assay comprises nucleic acid amplification by digital polymerase chain reaction, wherein, in the digital PCR, the sample is separated into partitions, wherein a PCR reaction is carried out in each partition individually, wherein partitions containing a target nucleotide sequence are amplified and produce a positive detection signal, while the partitions containing no target nucleotide sequence are not amplified and produce no detection signal; further comprising the steps of a) providing a dataset comprising a plurality of intensity values from a plurality of measurement samples; wherein said plurality of measurement samples are derived from a single biological test sample comprising nucleic acids; b) providing a predefined discrimination value for separating the intensity values into two different data categories; c) separating the plurality of intensity values into either one of the two different data categories by determining whether the individual values of the intensity values are above or below the predefined discrimination value; d) determining the cumulative probability function for all values in the data category above the discrimination value (Group A), and determining the cumulative probability function for all values in the data category below the discrimination value (Group B); e) obtaining a new discrimination value such that a ratio between the two cumulative probability functions corresponds to a predefined error factor; f) iterating steps c) to e) whereby the new discrimination value obtained in step e) after an iteration replaces the discrimination value of the previous iteration; and whereby the iteration is carried out until the compositions of data categories, (Group A, positives and Group B, negatives) remain constant or for a predetermined number of iterations; and g) automatically providing the new discrimination value obtained in step f) of the last iteration as threshold for allocating intensity values derived from a digital PCR assay from the biological sample comprising nucleic acids into either data category, Group A (positives) and Group B (negatives). Moreover, the invention provides a computer-readable medium comprising a computer program code that when run on a data processing device carries out the method of the invention as well as a device for carrying out the method of the invention.

### BACKGROUND

Research and/or diagnostic applications may have unequal sensitivity and specificity expectations. For certain applications a high sensitivity, i.e. a low false negative rate, is required, whereas for other applications a high specificity, i.e. few false positives, is needed. For examples, in blood screening applications high sensitivity is often regarded as key feature while for detection of certain mutation(s) specificity may be more important.

In particular, data sets derived from highly sensitive biological assays such as polymerase chain reaction (PCR), Flow Cytometry, nucleic acid hybridization, immunohistology and imaging often contain varying signals of positives and negatives and therefore show an overlapping signal distribution (sometimes also called rain).

Especially, when working in the low concentration range, e.g. less than 1% of partitions called positive, a false positive or false negative partition call can have a considerable impact by increasing or decreasing the reported concentration considerably above or below a threshold indicating presence. This may have a strong impact on the outcome and thus may be of harm. For example, a false medical report based on a false positive signal could be issued, indicating the presence of a certain DNA/mutation (which in reality is not present) and potentially affecting further treatment of the patient.

One of the most sensitive assays to detect an analyte of interest and, in particular, a certain nucleic acid, is digital PCR. In a digital PCR (dPCR) assay the sample is separated into a large number of partitions (aliquots) and the PCR reaction is carried out in each partition individually. This separation allows a more reliable collection and sensitive measurement of nucleic acid amounts and enables the quantitative detection of a (low copy) target DNA among a larger amount of (contaminating) DNA which may be present in a single biological sample. The partitions containing the target nucleotide sequence are amplified and produce a positive detection signal, while the partitions containing no target nucleotide sequence are not amplified and produce no detection signal. However, the large number of data points retrieved from a highly sensitive assay such as dPCR is challenging to analyze. Often, it is difficult to clearly discriminate between positive and negative signals as these may overlap. Particularly, in case the biological assay did not work perfectly, e.g. the annealing of primers in the PCR assay did not work as expected, a dataset with (largely) overlapping positive and negative signals may be present. Thus, it is crucial to define the right threshold to separate real positives from real negative signals to avoid false positive and/or false negative signals. For blood screening applications, i.e. detecting Hepatitis C Virus (HCV), high sensitivity is often regarded as key feature, i.e. the proportion of positives that are correctly identified as such (true positive rate). For other assays, e.g. the detection of a mutation in a cancer-related gene such as BRCA1, the proportion of negatives that are correctly identified as such (true negative rate) may be more important.

It would be very beneficial, if a predefined expectation regarding the relation of false positives to false negatives could be taken into account while a dataset from an assay is analyzed and an optimized threshold between positives and negatives is calculated automatically with regard to said predefined expectation. At the moment, no such method exists.

Currently, only simple threshold calculations, i.e. the discrimination of signals into positives and negatives based on an underlying assumption that the calls, e.g. the fluorescent signals of the dPCR partitions, are normally distributed, are available. The so calculated threshold may be manually adapted, e.g. by visual examination of the data, and a new threshold recalculated.

WO2014/153369 A1 discloses methods and systems for analyzing biological reaction systems. In particular, a method of chemically treating a surface of a substrate used in a biological reaction system to prevent biological molecules from adhering to the surface is disclosed. Further, the document relates to determining of fluorescence emission at certain locations within an image based on pixel intensity. Briefly, after determining the reaction site locations within the image, a determination of whether there is a fluorescent emission from a reaction site (positive call) or an absence of fluorescent emissions from a reaction site (negative call) can be determined based on intensities of the pixels. This may be accomplished by Otsu thresholding and various levels of thresholding may be used to discriminate between positives and negatives. Otsu thresholding, also referred to as Otsu's method, is an image processing method and is used to automatically perform clustering-based image thresholding or the reduction of a gray level image to a binary image. The algorithm assumes that the image contains two classes of pixels following bi-modal histogram (foreground pixels and background pixels), it then calculates the optimum threshold separating the two classes so that their combined spread (intra-class variance) is minimal, or equivalently (because the sum of pairwise squared distances is constant), so that their inter-class variance is maximal.

Various data analysis methods for biological assays are available. For example, WO 99/54724 A1 proposes a process for analyzing data obtained from nucleic acid hybridization arrays including microarrays and gene chips attempting to make precise and statistically valid inferences. However, a computer-implemented method for analyzing a dataset allowing for the precise allocation of a plurality of intensity values from a digital PCR assay into positives and negatives is not described.

WO2014/210559 A1 discloses methods and systems for visualizing data quality including receiving a plurality of data points related to fluorescent emissions values from a plurality of reaction sites. The fluorescent emission values include information for a first type of dye and a second type of dye. For example, from a first probe labeled with FAM (6-carboxyfluorescein) and a second probe labeled with VIC (4,7,2'-trichloro-7'-phenyl-6-carboxyfluorescein) as applied in PCR assays. Different dyes associated with the data may be displayed and positive and negative calls may be determined by a processing system. However, the assumption for all of these calls, i.e. positive for VIC, positive for FAM, positive for VIC and FAM, and negative calls, is that they should be uniformly distributed. However, in real life these calls are often clumped in certain areas rather than uniformly distributed. The user is provided with a tool to visualize the types of calls and to manually adjust the threshold on a well-by-well basis or across an entire plate so that the processor will then recalculate the results based on the manually adjusted threshold. However, this procedure is very time-consuming and does not take into account a pre-defined sensitivity and specificity expectation of a certain assay.

Moreover, various methods of threshold calculation as well as respective software packages for different biological assays and especially for the analysis of dPCR data are commercially available.

However, the current methods that allow discrimination of signals into positives and negatives are based on an underlying assumption that the calls, i.e. the fluorescent signals of the dPCR droplets, are normally distributed. For example, QuantaSoft^{™} from Bio-Rad Laboratories, Inc. for analysis of digital PCR data (derived e.g. from the QX200^{™} Droplet Digital^{™} system) will automatically calculate a threshold above which droplets are considered positive, based on the assumption that all of the calls are uniformly distributed.

Trypsteen et al, 2015 (Trypsteen W. et al., ddpcRquant: threshold determination for single channel droplet digital PCR experiments. Analytical and bioanalytical chemistry 407.19 (2015): 5827-5834) discloses a method of threshold calculation that does not make any assumptions about the distribution of the fluorescence readouts. Briefly, a threshold is estimated by modelling the extreme values in the negative droplet population using extreme value theory and taking shifts in baseline fluorescence between samples into account.

However, none of the available data analysis methods accounts for different sensitivity and/or specificity expectations and provides a fully automated solution for research and/or diagnostic applications. Moreover, the problems of instable thresholding for overlapping distributions of positive and negative signals and inadequate treatment of unequal effects of false-positives and false-negatives are not addressed by the current methods and/or devices.

The present invention is, therefore, concerned with the provision of an improved method and device for analyzing a data set to allocate intensity values derived from a digital PCR assay from the biological sample comprising nucleic acids into either one of two different data categories. This problem is solved by the embodiments characterized in the claims and described herein below.

The scope of the invention is defined by the appended claims.

### DESCRIPTION

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "particularly", "more particularly", "specifically", "more specifically", "typically", and "more typically" or similar terms are used in conjunction with additional / alternative features, without restricting alternative possibilities. Thus, features introduced by these terms are additional / alternative features and are not intended to restrict the scope of the claims in any way. The disclosure may, as the skilled person will recognize, be performed by using alternative features. Similarly, features introduced by "in an embodiment of the invention" or similar expressions are intended to be additional / alternative features, without any restriction regarding alternative embodiments of the disclosure, without any restrictions regarding the scope of the disclosure and without any restriction regarding the possibility of combining the features introduced in such way with other additional / alternative or non-additional / alternative features of the disclosure.

The present disclosure relates to a method for analyzing a dataset comprising a step of performing an analyzing assay and retrieving intensity values from said assay, wherein said analyzing assay comprises nucleic acid amplification by digital polymerase chain reaction (dPCR), wherein, in the digital PCR, the sample is separated into partitions, wherein a PCR reaction is carried out in each partition individually, wherein partitions containing a target nucleotide sequence are amplified and produce a positive detection signal, while the partitions containing no target nucleotide sequence are not amplified and produce no detection signal; further comprising the steps of:
a) providing a dataset comprising a plurality of intensity values from a plurality of measurement samples; wherein said plurality of measurement samples are derived from a single biological test sample comprising nucleic acids;
b) providing a predefined discrimination value for separating the intensity values into two different data categories;
c) separating the plurality of intensity values into either one of the two different data categories by determining whether the individual values of the intensity values are above or below the predefined discrimination value;
d) determining the cumulative probability function for all values in the data category above the discrimination value (Group A) and determining the cumulative probability function for all values in the data category below the discrimination value (Group B);
e) obtaining a new discrimination value such that a ratio between the two cumulative probability functions corresponds to a predefined error factor;
f) iterating steps c) to e)
   - whereby the new discrimination value obtained in step e) after an iteration replaces the discrimination value of the previous iteration; and
   - whereby the iteration is carried out until the compositions of data categories (Group A, positives and Group B, negatives) remain constant or for a predetermined number of iterations; and
g) automatically providing the new discrimination value obtained in step f) of the last iteration as threshold for allocating intensity values derived from a digital PCR assay from the biological sample comprising nucleic acids into either data category, Group A, positives and Group B, negatives.

The term "dataset" as used herein refers to a collection of data comprising a plurality of measurement data derived from a plurality of measurement samples. The dataset is derived from a biological assay that allows absolute quantification. Said dataset is a dataset retrieved by performing a digital PCR (dPCR), particularly a droplet digital PCT (ddPCR). ddPCR is a method for performing digital PCR that is based on water-oil emulsion droplet technology. Here, a single test sample, for example a biological sample comprising nucleic acids, is fractionated into about 20,000 droplets, and PCR amplification of the template molecules occurs in each individual droplet. ddPCR technology uses reagents and workflows similar to those used for most standard quantitative PCR techniques, e.g. TaqMan^{™} probe-based assays. Thus, said plurality of measurement samples may relate to said dPCR droplets, where each droplet emits a strong, low or no fluorescent signal, resulting in a plurality of measurement data (i.e. many data points relating to fluorescence intensity values). The dataset retrieved from an analyzing assay, such as a dPCR assay, may also be pre-processed, for example the dataset to be used in the methods of the present disclosure may only contain part of the original data derived from an analyzing assay. Particularly, said plurality of measurement data relates to more than 100, more than 1,000, more than 2,000, more than 5,000, more than 10,000, more than 20,000 or more than 50,000 data points.

The measurement data are intensity values derived from the measurement sample and which are indicative of at least one chemical or physical property of said measurement sample and, in particular, its ingredients. Typically, said measurement data are intensity values which can be correlated quantitatively to the amount of at least one component comprised in the measurement sample, e.g., a biomolecule such as a nucleic acid or an organism such as a cell present in the measurement sample. More typically, said intensity values are intensity values of fluorescence, chemiluminescence, radiation or colorimetric changes. For example, intensity values of fluorescence may be emitted during a nucleic acid hybridization assay or Flow Cytometry. Chemiluminescence, radiation or colorimetric changes may be detected in immunohistology and imaging assays.

Said measurement data are intensity values derived from a digital PCR assay. As also explained elsewhere herein, a plurality of measurement data (e.g. fluorescence intensity data) is derived from a plurality of measurement samples (e.g. ddPCR droplets) derived from a single test sample, a biological sample comprising nucleic acids.

The term "test sample" as used herein refers to any type of sample from any source to be analyzed by the method of the present disclosure. Typically, the test sample is a liquid sample that can be partitioned into measurement samples. Moreover, the sample can be either an artificial sample, i.e. a sample which is artificially composed or results from an artificially initiated chemical reaction, or can be a naturally occurring sample or a sample derived from a natural source, e.g., a biological, food or environmental sample. Said test sample is a biological sample. Biological samples include samples derived from an organism, typically a mammalian organism, such as body fluid samples, e.g., blood, or tissue samples, samples containing bacterial and/or viral particles as well as food samples such as fruit extracts, meat or cheese. Typical test samples include liquid samples such as samples of body fluids like blood, plasma, serum or urine, fruit juices and solid samples such as tissue samples or food extracts. Also, said test sample comprises nucleic acids and/or cells. It is known to those skilled in the art that a test sample, in particular, a biological test sample comprising nucleic acids and/or cells, may have been pre-processed, for example, by isolating cells or DNA from a tissue or DNA from cells. Means and methods for isolation and/or treatment of biological samples are well known in the art and include, for example, the separation of blood samples by centrifugation, the use of coated magnetic microbeads or the use of solid phase binding materials for rapidly isolating nucleic acids from samples. Further, the test sample is a single test sample from which the plurality of measurement samples can be obtained. For example, said single test sample may be a biological sample, such as a blood sample comprising nucleic acids that is subsequently analyzed by ddPCR as explained elsewhere herein in detail, i.e. a single sample comprising nucleic acids is partitioned into around 20,000 droplets which correspond to said plurality of measurement samples and the florescence intensity of these partitions is recorded thereby providing a plurality of measurement data.

The term "predefined discrimination value" as used herein, also referred to as initial threshold, relates to a value that is used for separating the measurement data into two different data categories, e.g., positives and negatives. All values in the data category above the predefined discrimination value are considered as, e.g., positives (Group A) and all values in the data category below the predefined discrimination value are considered as, e.g., negatives (Group B). The predefined discrimination value (initial threshold) may be any value within the minimum and the maximum value of the dataset. Typically, said predefined discrimination value is the median, arithmetic mean or any percentile between the 15^{th} and the 85^{th} percentile of the measurement data.

Means and methods for the discrimination of data into different categories as well as the statistical analysis of a dataset, e.g. calculating mean or median values, normal distributions and cumulative probability functions, are known to the person skilled in the art can also be found in standard text books of statistics.

According to the present disclosure, the ratio between two cumulative probability functions for Group A (positives) and Group B (negatives) shall be optimized with regard to a predefined error factor. This means that the ratio between the two cumulative probability functions shall correspond to a predefined expectation concerning the presence of false positives or false negatives, i.e. correspond to the predefined error factor as explained elsewhere herein. Thereby a new discrimination value, i.e. a optimized threshold, is obtained. In particular, obtaining a new discrimination value may, typically, comprise the following:
- determining the intersection point between the cumulative probability functions of positives (Group A) and negatives (Group B)
- calculating the probability of said intersection point deriving two new cumulative probability functions
- multiplying or dividing said new cumulative probability functions by the square root of the predefined error factor and calculating two approximation points using inverse cumulative probability functions;
- interpolating the probability density function ratio of the approximation points thereby obtaining a new discrimination value.

Means and methods to perform the above listed calculations are known to those skilled in the art and can also be found in standard textbooks relating to statistical analysis and probability calculation. Respective calculations can also be performed using computer programs such as Microsoft^{®} Exel or MATLAB^{®}. For example, the inverse cumulative distribution function specifies, for a given probability in the probability distribution of a random variable, the value at which the probability of the random variable is less than or equal to the given probability. An interpolation refers to a method of constructing new data points within the range of a discrete set of known data points. Interpolation methods include, for example, linear interpolation, logarithmic interpolation, polynomial interpolation, spline interpolation, interpolation via Gaussian processes, interpolation by rational functions and multivariate interpolation. According to the present disclosure, the interpolation, e.g. interpolation of the probability density function ratio of the approximation points, is typically done logarithmically.

The term "new discrimination value" as used herein, also referred to as new, better or optimized threshold, relates to a discrimination value that takes the predefined error factor into account as explained elsewhere herein. The new discrimination value is calculated as described above and derived after each iteration cycle. After carrying out a certain predetermined number of iterations until the compositions of the data categories, i.e. the number of data points in Group A and Group B, remain constant, the (final) new discrimination value is used for allocating measurement data from a test measurement sample into either data category. The measurement data according to the invention are intensity values as described elsewhere herein. It will be understood that the iteration process can also be carried out as long as required until the compositions of the data categories, i.e. the number of data points in Group A and Group B, remain constant.

The term "error factor" as used herein refers to a predefined expectation regarding the presence of false positives and false negatives. In other words, the error factor relates to the desired quality of the result. The error a factor accounts for a pre-defined probability bias, e.g. fewer false positives are desired. According to the present disclosure, the ratio between the two cumulative probability functions of Group A (positives) and Group B (negatives) shall correspond to a predefined expectation concerning the presence of false positives or false negatives, i.e. to the predefined error factor. For example, a high error factor may relate to the ratio of false negatives to false positives while a low error factor may relate to the ratio of false positives to false negatives. As mentioned above, the definition of error factor depends on the desired sensitivity and specificity. The error factor may be chosen differently for each assay and pre-set at the beginning of the analysis of the dataset, i.e. the automatic calculation of a new discrimination factor with regard to the pre-set error factor in accordance with the present disclosure. A mentioned above, data sets derived from highly sensitive biological assays such as a digital PCR often show an overlapping signal distribution and a false positive or false negative call can have a considerable impact by increasing or decreasing the reported concentration considerably above or below a threshold indicating presence. For example, a false medical report based on a false positive signal could be issued, indicating the presence of a certain DNA/mutation which in reality is not present. Thus, the error factor may be chosen for each assay individually accounting for the expectation concerning the sensitivity and specificity of the result, i.e. fewer false positives or fewer false negatives, respectively.

Typically, the error factor is below or equal to 100, below or equal to 80, below or equal to 60, below or equal to 50, below or equal to 40, below or equal to 30, below or equal to 20 or below or equal to 10.

According to the method of the present disclosure, the square root of the error factor is typically used. As mentioned also elsewhere herein, typically, the intersection point of the cumulative probability functions of the negative tail of the positive group and the positive tail of the negative group is calculated followed by the calculation of the probability of this point deriving two new probabilities yielding two new cumulative probability functions. Then the error factor is used as a pre-set factor between false positive and false negative rate. The probabilities are then multiplied or divided, respectively, divided by the square root of the error factor and with the help of inverse cumulative probability functions two approximation points for the new positive negative threshold are calculates. The probability density function ratio of these approximation points is then interpolated, preferably logarithmically, to get a new discrimination value, i.e. a better estimate for the new positive negative threshold.

The term "iterating" or "iteration" as used herein refers to repeating defined steps of the method in a cyclic manner wherein each new iteration cycle starts with the outcome of the previous iteration cycle in order to approach a desired result. The iteration characterized by repeating certain steps, i.e. steps c) to i) of the method according to the present disclosure, will lead to a new discrimination value as described elsewhere herein. According to the present disclosure, the iteration starts with separating the plurality of measurement data into either one of the two different data categories by determining whether the individual values of the measurement data are above (Group A, positives) or below (Group B, negatives) the predefined discrimination value, preferably the median, arithmetic mean or any percentile between the 15^{th} and the 85^{th} percentile of the measurement data, as explained elsewhere herein. Then the resulting two groups of positives (Group A) and negatives (Group B) are analyzed, i.e. the ratio of the two cumulative probability functions (Group A and Group B) is optimized with regard to a predefined error factor thereby obtaining a new discrimination value as explained elsewhere herein. The iteration shall take place until the compositions of data categories (Group A and Group B) remain constant or for a predetermined number of iterations. Typically, the predetermined number of iterations is any number between 5 and 20, between 5 and 15, between 5 and 12 or between 5 and 10.

It will be understood that the method of the present disclosure may include additional steps and is a computer-implemented method. Typically, the method of the present disclosure is an automated method, meaning that at least one step and more commonly, more than one step, is automated.

The method according to the present invention further comprises the step of performing an analyzing assay and retrieving the measurement data from said assay. An analyzing assay may be any assay, typically a biological assay that provides a plurality of measurement data from a plurality of measurement samples. The analyzing assay comprises nucleic acid amplification by polymerase chain reaction (PCR). PCR is well known in the art and describes a technique used to amplify DNA. PCR typically includes the steps of subjecting double stranded nucleic acids in a reaction mixture to reversibly denaturing conditions ("denaturing step"), e.g. by heating above the melting temperature of the double stranded nucleic acids in an effort to disrupt the hydrogen bonds between complementary bases, yielding single-stranded DNA molecules, annealing a primer to each of the single stranded nucleic acids ("annealing step"), and extending the primers by attaching mononucleotides to the ends of said primers using the sequence of the single stranded nucleic acid as a template for newly formed sequences ("extension/elongation step"). The processes of denaturation, annealing and elongation constitute of one cycle. The reaction cycle is repeated as many times as desired, for example between 10 and 100 times. The term "PCR" includes quantitative (qPCR) and qualitative PCR as well as any kind of PCR variant such as Asymmetric PCR, Allele-specific PCR, Assembly PCR, Dial-out PCR, Digital PCR (dPCR), Helicase-dependent amplification, Hot start PCR, In silico PCR, Intersequence-specific PCR (ISSR), Inverse PCR, Ligation-mediated PCR, Methylation-specific PCR (MSP), Miniprimer PCR, Multiplex ligation-dependent probe amplification (MLPA), Nanoparticle-Assisted PCR (nanoPCR), Nested PCR, Reverse Transcription PCR (RT-PCR), qRT-PCR, Solid Phase PCR, Thermal asymmetric interlaced PCR (TAIL-PCR), Touchdown PCR (Step-down PCR) and Universal Fast Walking. In the method according to the invention described herein, the PCR is digital PCR (dPCR).

Digital PCR can be used to directly quantify and clonally amplify nucleic acid strands including DNA, cDNA or RNA. The key difference between dPCR and traditional PCR lies in the method of measuring nucleic acids amounts, i.e. measurement of one fluorescence measurement (classical PCR) versus a plurality, e.g., thousands, of distinct fluorescence measurements (dPCR). Classical PCR carries out one reaction per single sample. Although, dPCR also carries out a single reaction within a sample, the sample is however separated into a large number of partitions and the reaction is carried out in each partition individually. For separation of a single sample, micro well plates, capillaries, oil emulsion, and arrays of miniaturized chambers with nucleic acid binding surfaces can be used. Droplet Digital PCR (ddPCR) is a method for performing digital PCR (dPCR) that is based on water-oil emulsion droplet technology. A single test sample is fractionated into droplets, typically, about 20,000 nanoliter-sized droplets, and PCR amplification of the template molecules occurs in each individual droplet. ddPCR technology uses reagents and workflows similar to those used for most standard quantitative PCR techniques, e.g. TaqManTM probe-based assays which consist of template DNA (or RNA), Fluorescence-Quencher probes, primers, and a PCR master mix. The PCR solution is divided into smaller reactions (droplets) that are then made to run PCR individually. After multiple PCR amplification cycles, the samples are checked for the presence or absence of fluorescence (with a binary code of "0" and "1"). Typically, the fraction of fluorescing droplets is recorded by an instrument and analyzed by instrument software. Due to separation/partitioning of the sample and by assuming that that the molecule population follows the Poisson distribution, the distribution of target molecule within the sample can be approximated allowing for a quantification of the target strand in the PCR product.

It is known in the art that further methods and algorithms may be applied to a PCR dataset and that analysis of the large number of data points retrieved from dPCR may be challenging, for example due to overlapping distributions of positive and negative signals and inadequate treatment of unequal effects of false-positives and false-negatives. However, these obstacles are overcome by the method according to the present disclosure. Advantageously, the method of the present disclosure, in general, solves the problem of instable thresholding for overlapping distributions of positive and negative signals and inadequate treatment of unequal effects of false-positives and false-negatives. By employing an iterative procedure the optimal threshold level between positive and negative calls with regard to a predefined error factor can be determined, providing a fully automated solution for, e.g., research and/or diagnostic applications such as the aforementioned dPCR applications.

The present disclosure further relates to a computer-readable medium comprising computer program code that when run on a data processing device carries out the method of the present invention. Thus, the disclosure encompasses a computer program including computer-executable instructions for performing the method according to the present invention in one or more of the embodiments enclosed herein when the program is executed on a computer or computer network. Specifically, the computer program may be stored on a data carrier. Thus, specifically, one, more than one or even all of method steps a) to g) as indicated above may be performed by using a computer or a computer network, preferably by using a computer program. The disclosure also encompasses and proposes a computer program product having program code means, in order to perform the method according to the present invention in one or more of the embodiments enclosed herein when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Preferably, referring to the computer-implemented aspects of the disclosure, one or more of the method steps or even all of the method steps of the method according to one or more of the embodiments disclosed herein may be performed by using a computer or computer network. Thus, generally, any of the method steps including provision and/or manipulation of data, e.g. separating data or optimizing the ratio of two cumulative probability functions, may be performed by using a computer or computer network. Generally, these method steps may include any of the method steps, typically except for method steps requiring manual work, such as providing the samples and/or certain aspects of performing the actual measurements, e.g. performing a dPCR analyzing assay and retrieving the measurement data from said assay.

Specifically, the present disclosure further encompasses:
- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer script, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

The present disclosure encompasses a device for carrying out the method according to the present invention comprising
a) a data storage unit comprising a plurality of measurement data from a plurality of measurement samples; and
b) a data processing unit having tangibly embedded a computer program code carrying out the method of the present invention.

Means and methods to record, store and process data are well known in the art. A data storage unit according to the present disclosure may be any means capable of storing data such as a hard drive, flash drive, CD-R or DVD-R. A data processing unit may be any platform that comprises a computer program code that is able to carry out the method of the present disclosure.

Preferably, the device for carrying out the method according to the present disclosure further comprises a measurement unit capable of obtaining the measurement data from the measurement samples. For example, the measurement unit may be a detector capable of obtaining fluorescence intensity values from the droplets of a ddPCR assay as explained elsewhere herein.

Moreover, the device may, typically, further comprise an analyzing unit capable of carrying out an analyzing assay, said analyzing assay comprises nucleic acid amplification by digital polymerase chain reaction (dPCR) as explained elsewhere herein in detail.

The device may also, typically, comprise an output unit for providing the new discrimination value as threshold, preferably, on a graphical display. Suitable output units and graphical displays are well known in the art and include for example an output device incorporating a cathode ray tube on which both line drawings and text can be displayed. Such a graphical display may also be used in conjunction with a light pen to input or reposition data. Moreover, the output unit shall preferably, e.g. via a graphical display, further allocate additional information on said threshold.

The above explanations and definitions of the terms apply throughout the specification.

### FIGURES

**Figure 1** shows an example for automated Positive/Negative Calling. The settings were as follows: Settings: Initial Guess: 0.8 * Min + 0.2 Max: 1261; Probability bias = 10 (less false positives); Threshold after 8 iterations: 709
**Figure 2** shows a further example for automated Positive/Negative Calling: The settings were as follows: Initial Guess: 0.8 * Min + 0.2 Max: 1824; Probability bias = 10 (less false positives). Threshold after 10 iterations: 1846

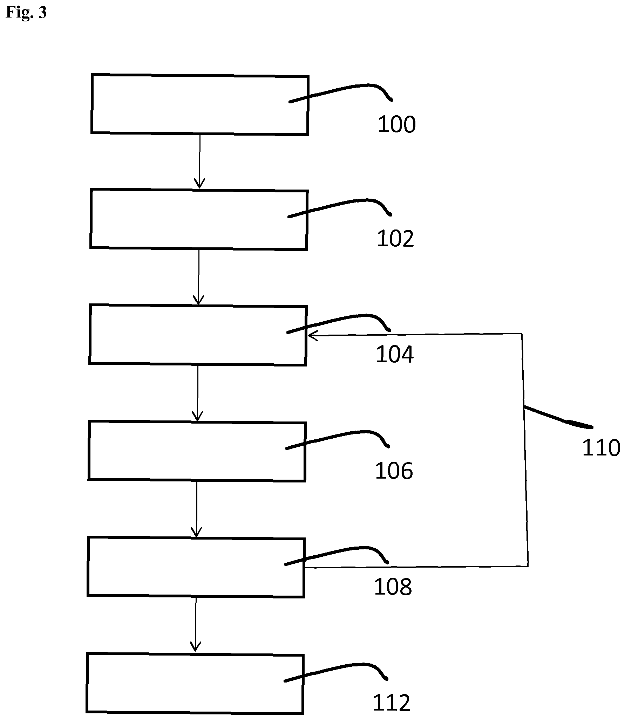

**Figure 3** shows a schematic presentation of a method for analyzing a dataset according to the present disclosure.

### EXAMPLES

The following Examples shall illustrate the disclosure. They shall, by no means, construed as limiting the scope for which protection is sought.

### Example: Algorithm for Positive/Negative Calling

In a first step, an initial guess for threshold is made. Subsequently, the distribution parameter of positives and negatives is determined automatically. To this end, the threshold is set so that the derived probabilities of false-positives and false-negatives fulfill a pre-set probability ratio. An iterative application is carried out next to improve threshold level until ratio of positives and negatives remain constant. The concept here consists in performing an iterative procedure to determine the optimal threshold level between positive and negative call (compared to just use a pre-set cutoff). The iteration starts with an initial threshold, e.g. the (weighted) mean of the maximal and minimal signal level. Then the resulting two groups of positives and negatives are analyzed. Based on an adequate pre-set subset of the data e.g. Percentile range 5% at inner to 99% at outer range (for possibly overlapping distributions) the theoretical cumulative probability distribution is calculated for each group (positives and negatives), e.g. the error function for normal distributions. In a next step, the intersection point of the cumulative probability functions of the negative tail of the positive group and the positive tail of the negative group is calculated. Afterwards, the probability of this point is calculated. Subsequently, a pre-set factor between false positive and false negative rate is used. The probabilities are to be divided or multiplied with by the square root of this factor and with the help of the inverse cumulative probability functions two approximation points for the new positive negative threshold are calculated. The probability density function ratio of these points is logarithmically interpolated to get a good estimate for the new positive negative threshold. This iteration is repeated until the number of positives and negatives remains constant or at maximum a pre-set times, e.g. 10 times.

The aforementioned algorithm can be implemented by the following program instructions:

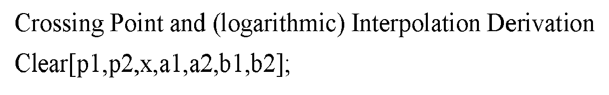

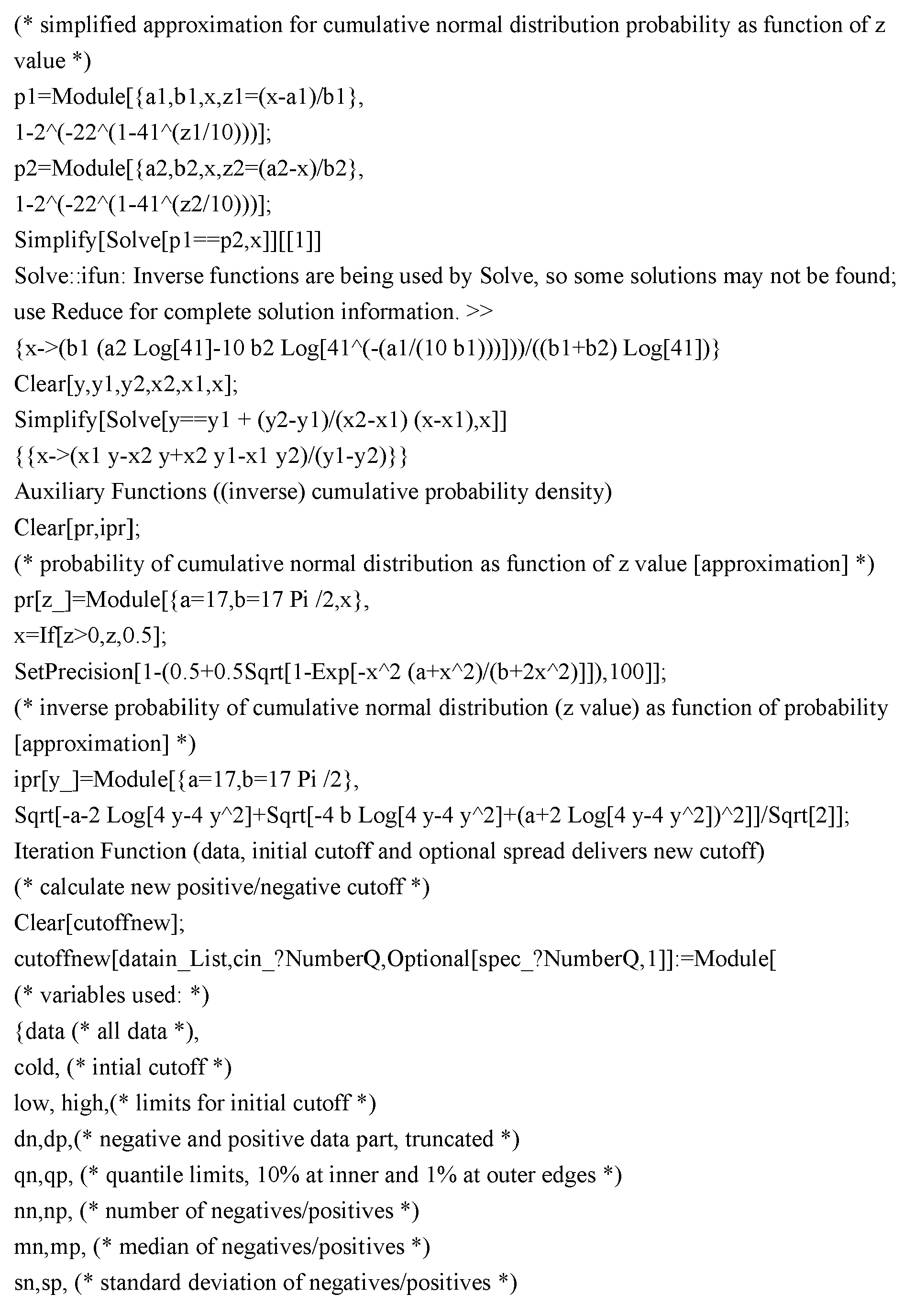

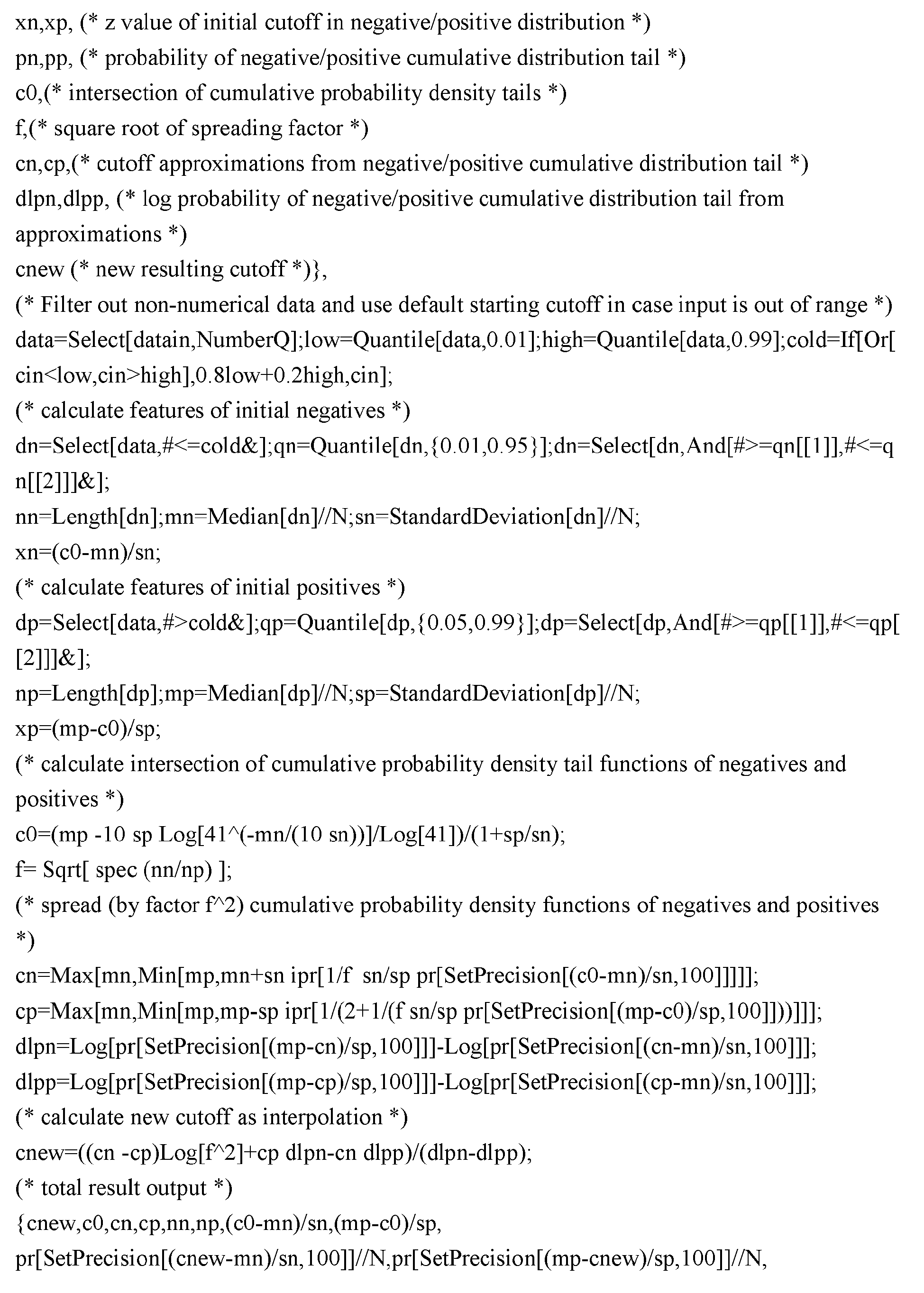

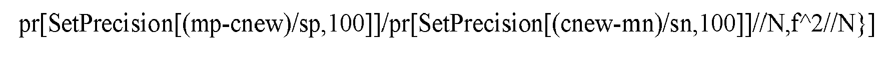

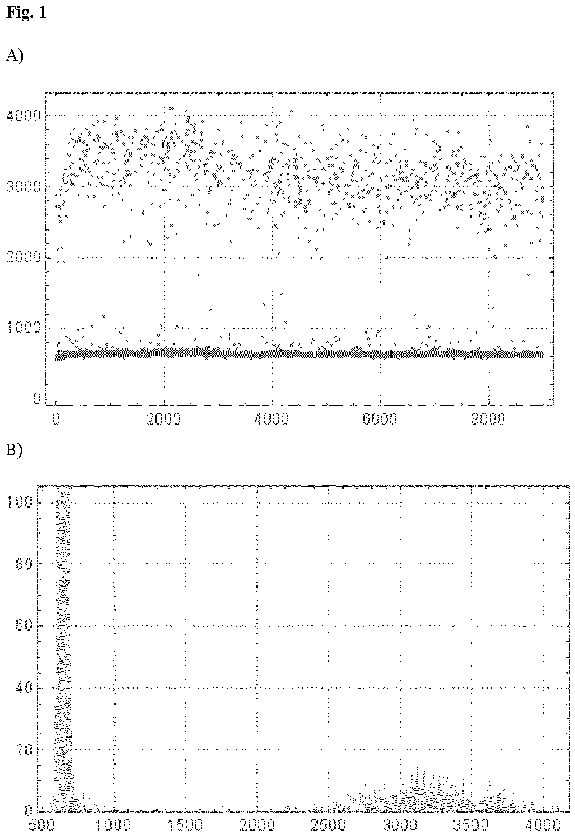

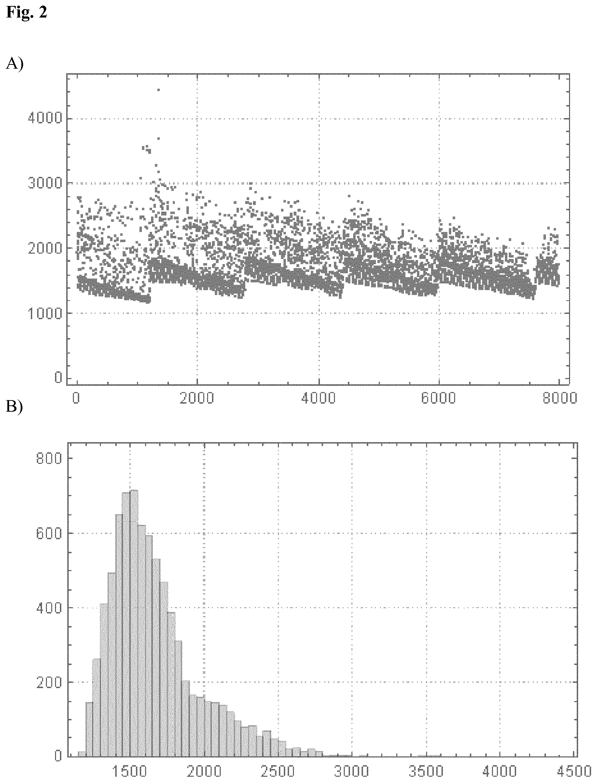

Using this program to implement the algorithm, data could be analyzed as shown in Figures 1 and 2. The settings in the experiment analyzed in Figure 1 were as follows: Settings: Initial Guess: 0.8 * Min + 0.2 Max: 1261; Probability bias = 10 (less false positives); Threshold after 8 iterations: 709. The settings in the experiment analyzed in Figure 2 were as follows: Initial Guess: 0.8 * Min + 0.2 Max: 1824; Probability bias = 10 (less false positives). Threshold after 10 iterations: 1846.

### List of reference numbers

- 100:: provision of a dataset comprising a plurality of measurement data from a plurality of measurement samples
- 102:: provision of a predefined discrimination value for separating the measurement data into two different data categories
- 104:: separation of the plurality of measurement data into either one of the two different data categories by determining whether the individual values of the measurement data are above or below the predefined discrimination value
- 106:: Determination of the cumulative probability function for all values in the data category above the discrimination value (Group A) and determining the cumulative probability function for all values in the data category below the discrimination value (Group B)
- 108:: Optimizing the ratio of the two cumulative probability functions determined in step d) with regard to a predefined error factor, thereby obtaining a new discrimination value
- 110:: Iteration of steps 104 to 108 whereby the new discrimination value obtained in step e) after an iteration replaces the discrimination value of the previous iteration and whereby the iteration is carried out until the compositions of data categories (Group A and Group B) remain constant or for a predetermined number of iterations
- 112:: Provision of the new discrimination value obtained in step 110 of the last iteration as threshold for allocating measurement data from a test measurement sample into either data category

### List of cited references

WO 99/54724 A1;
WO2014/153369 A1;
WO2014/210559 A1;
Trypsteen W. et al., ddpcRquant: threshold determination for single channel droplet digital PCR experiments. Analytical and bioanalytical chemistry 407.19 (2015): 5827-5834.

## Claims

1. A computer-implemented method for analyzing a dataset, the method comprising a step of performing an analyzing assay and retrieving intensity values from said assay, wherein said analyzing assay comprises nucleic acid amplification by digital polymerase chain reaction,
wherein, in the digital PCR, the sample is separated into partitions, wherein a PCR reaction is carried out in each partition individually, wherein partitions containing a target nucleotide sequence are amplified and produce a positive detection signal, while the partitions containing no target nucleotide sequence are not amplified and produce no detection signal;
further comprising the steps of:
a) providing a dataset comprising a plurality of intensity values from a plurality of measurement samples; wherein said plurality of measurement samples are derived from a single biological test sample comprising nucleic acids;
b) providing a predefined discrimination value for separating the intensity values into two different data categories;
c) separating the plurality of intensity values into either one of the two different data categories by determining whether the individual values of the intensity values are above or below the predefined discrimination value;
d) determining the cumulative probability function for all values in the data category above the discrimination value, Group A, and determining the cumulative probability function for all values in the data category below the discrimination value, Group B;
e) obtaining a new discrimination value such that a ratio between the two cumulative probability functions corresponds to a predefined error factor;
f) iterating steps c) to e)
- whereby the new discrimination value obtained in step e) after an iteration replaces the discrimination value of the previous iteration; and
- whereby the iteration is carried out until the compositions of data categories, Group A positives and Group B negatives, remain constant or for a predetermined number of iterations; and
g) automatically providing the new discrimination value obtained in step f) of the last iteration as threshold for allocating intensity values derived from a digital PCR assay from the biological sample comprising nucleic acids into either data category, Group A positives and Group B negatives.

2. The method of claim 1, wherein said intensity values are intensity values of fluorescence, chemiluminescence, radiation or colorimetric changes.

3. The method of any one of claims 1 to 2, wherein said predefined discrimination value for separating the intensity values into two different data categories is the median, arithmetic mean or any percentile between the 15^{th} and the 85^{th} percentile of the intensity values.

4. The method of any one of claims 1 to 3, wherein step e) comprises:
i. determining the intersection point between the cumulative probability functions of step d);
ii. calculating the probability of said intersection point deriving two new cumulative probability functions
iii) multiplying or dividing said new cumulative probability functions by the square root of the predefined error factor and calculating two approximation points using inverse cumulative probability functions;
v) interpolating the probability density function ratio of the approximation points thereby obtaining a new discrimination value.

5. The method of any one of claims 1 to 4, wherein the error factor is below or equal to 100, below or equal to 80, below or equal to 60, below or equal to 50, below or equal to 40, below or equal to 30, below or equal to 20 or below or equal to 10.

6. The method of any one of claims 1 to 5, wherein said predetermined number of iterations is any number between 5 and 20, between 5 and 15, between 5 and 12 or between 5 and 10.

7. The method of any one of claims 1 to 6, further comprising the following step:
h) obtaining a constant number of data points in Group A and Group B.

8. The method of any one of claims 1 to 7, wherein the error factor is below or equal to 10.

9. The method of any one of claims 1 to 8, wherein the error factor is a predefined expectation regarding the presence of false positives and false negatives.

10. The method of any one of claims 1 to 9, further comprising the following step: i) allocating the measurement data into either one of the two different data categories.

11. The method of any one of claims 1 to 10, wherein the dataset is a dataset retrieved by performing droplet digital PCR.

12. A computer-readable medium comprising a computer program code that when run on a data processing device carries out the method of any one of claims 1 to 11.

13. A device for carrying out the method of any one of claims 1 to 11 comprising:
a) a data storage unit comprising a plurality of intensity values from a plurality of measurement samples; and
b) a data processing unit having tangibly embedded a computer program code carrying out the method of any one of claims 1 to 11.

14. The device of claim 13, wherein said device further comprises a measurement unit capable of obtaining the intensity values from the measurement samples and, preferably, an analyzing unit capable of carrying out an analyzing assay.

## Patentansprüche

1. Computerimplementiertes Verfahren zum Analysieren eines Datensatzes, wobei das Verfahren einen Schritt des Durchführens eines Analyseassays und Abrufen von Intensitätswerten aus dem Assay umfasst, wobei der Analyseassay Nukleinsäureamplifikation durch digitale Polymerasekettenreaktion umfasst,
wobei die Probe bei der digitalen PCR in Teilproben geteilt wird, wobei eine PCR-Reaktion individuell in jeder Teilprobe ausgeführt wird, wobei Teilproben, die eine Ziel-Nukleotidsequenz enthalten, amplifiziert werden und ein positives Detektionssignal erzeugen, während die Teilproben, die keine Ziel-Nukleotidsequenz enthalten, nicht amplifiziert werden und kein Detektionssignal erzeugen;
ferner umfassend die folgenden Schritte:
a) Bereitstellen eines Datensatzes, der eine Mehrzahl von Intensitätswerten einer Mehrzahl von Messproben umfasst,; wobei die Mehrzahl von Messproben von einer einzigen biologischen Testprobe, die Nukleinsäuren umfasst, abgeleitet wird;
b) Bereitstellen eines vordefinierten Diskriminierungswertes zum Einteilen der Intensitätswerte in zwei verschiedene Datenkategorien;
c) Einteilen der Mehrzahl von Intensitätswerten in eine der zwei verschiedenen Datenkategorien durch Bestimmen, ob die individuellen Werte der Intensitätswerte über oder unter dem vordefinierten Diskriminierungswert sind;
d) Bestimmen der kumulativen Wahrscheinlichkeitsfunktion für alle Werte in der Datenkategorie über dem Diskriminierungswert, Gruppe A, und Bestimmen der kumulativen Wahrscheinlichkeitsfunktion für alle Werte in der Datenkategorie unter dem Diskriminierungswert, Gruppe B;
e) Erhalten eines neuen Diskriminierungswerts, derart dass ein Verhältnis zwischen den zwei kumulativen Wahrscheinlichkeitsfunktionen einem vordefinierten Fehlerfaktor entspricht;
f) Wiederholen der Schritte c) bis e)
- wobei der in Schritt e) erhaltene neue Diskriminierungswert nach einer Iteration den Diskriminierungswert der vorherigen Iteration ersetzt; und
- wobei die Iteration durchgeführt wird, bis die Zusammensetzungen der Datenkategorien, Gruppe A, positive Ergebnisse, und Gruppe B, negative Ergebnisse, konstant bleiben, oder für eine vorbestimmte Anzahl an Iterationen; und
g) automatisches Bereitstellen des in Schritt f) erhaltenen neuen Diskriminierungswerts der letzten Iteration als Schwelle zum Zuweisen von Intensitätswerten, die aus einem digitalen PCR-Assay von der Nukleinsäuren umfassenden biologischen Probe abgeleitet sind, zu jeder Datenkategorie, Gruppe A, positive Ergebnisse, und Gruppe B, negative Ergebnisse.

2. Verfahren nach Anspruch 1, wobei die Intensitätswerte Intensitätswerte von Fluoreszenz, Chemolumineszenz, Strahlung oder kolorimetrischen Änderungen sind.

3. Verfahren nach einem der Ansprüche 1 bis 2, wobei der vordefinierte Diskriminierungswert zum Einteilen der Intensitätswerte in zwei verschiedene Datenkategorien der Median, das arithmetische Mittel oder ein beliebiges Perzentil zwischen dem 15. und dem 85. Perzentil der Intensitätswerte ist.

4. Verfahren nach einem Ansprüche 1 bis 3, wobei Schritt e) umfasst:
i. Bestimmen des Schnittpunkts zwischen den kumulativen Wahrscheinlichkeitsfunktionen von Schritt d) ;
ii. Berechnen der Wahrscheinlichkeit des Schnittpunkts zum Ableiten von zwei neuen kumulativen Wahrscheinlichkeitsfunktionen;
iii) Multiplizieren oder Dividieren der neuen kumulativen Wahrscheinlichkeitsfunktionen mit der bzw. durch die Quadratwurzel des vordefinierten Fehlerfaktors und Berechnen zweier Näherungspunkte unter Verwendung inverser kumulative Wahrscheinlichkeitsfunktionen;
v) Interpolieren des Wahrscheinlichkeitsdichtefunktionsverhältnisses der Näherungspunkte, um dadurch einen neuen Diskriminierungswert zu erhalten.

5. Verfahren nach einem der Ansprüche 1 bis 4, wobei der Fehlerfaktor unter oder gleich 100, unter oder gleich 80, unter oder gleich 60, unter oder gleich 50, unter oder gleich 40, unter oder gleich 30, unter oder gleich 20 oder unter oder gleich 10 ist.

6. Verfahren nach einem der Ansprüche 1 bis 5, wobei die vorbestimmte Anzahl von Iterationen eine beliebige Anzahl zwischen 5 und 20, zwischen 5 und 15, zwischen 5 und 12 oder zwischen 5 und 10 ist.

7. Verfahren nach einem der Ansprüche 1 bis 6, ferner umfassend den folgenden Schritt:
h) Erhalten einer konstanten Anzahl von Datenpunkten in Gruppe A und Gruppe B.

8. Verfahren nach einem der Ansprüche 1 bis 7, wobei der Fehlerfaktor unter oder gleich 10 ist.

9. Verfahren nach einem der Ansprüche 1 bis 8, wobei der Fehlerfaktor ein vordefinierter Erwartungswert hinsichtlich des Vorhandenseins von falsch positiven Ergebnissen und falsch negativen Ergebnissen ist.

10. Verfahren nach einem der Ansprüche 1 bis 9, ferner umfassend den folgenden Schritt: i) Zuweisen der Messdaten zu einer der zwei verschiedenen Datenkategorien.

11. Verfahren nach einem der Ansprüche 1 bis 10, wobei der Datensatz ein Datensatz ist, der durch Durchführen von digitaler Tröpfchen-PCR abgerufen wird.

12. Computerlesbares Medium, das einen Computerprogrammcode speichert, der bei Ausführung auf einer Datenverarbeitungsvorrichtung das Verfahren nach einem der Ansprüche 1 bis 11 durchführt.

13. Vorrichtung zum Durchführen des Verfahrens nach einem der Ansprüche 1 bis 11, umfassend:
a) eine Datenspeichereinheit, die eine Mehrzahl von Intensitätswerten von einer Mehrzahl von Messproben umfasst; und
b) eine Datenverarbeitungsvorrichtung mit einem konkret eingebetteten Computerprogrammcode, der das Verfahren nach einem der Ansprüche 1 bis 11 durchführt.

14. Vorrichtung nach Anspruch 13, wobei die Vorrichtung ferner eine Messeinheit, die zum Erhalten der Intensitätswerte aus den Messproben imstande ist, und vorzugsweise eine Analyseeinheit umfasst, die zum Durchführen eines Analyseassays imstande ist.

## Revendications

1. Procédé mis en œuvre par ordinateur pour analyser un ensemble de données, le procédé comprenant une étape de réalisation d'un dosage analytique et récupération de valeurs d'intensités à partir dudit dosage, dans lequel ledit dosage analytique comprend une amplification d'acides nucléiques par amplification en chaîne par polymérase numérique,
dans lequel, dans la PCR numérique, l'échantillon est séparé en partitions, dans lequel une réaction de PCR est réalisée dans chaque partition individuellement, dans lequel les partitions contenant une séquence nucléotidique cible sont amplifiées et produisent un signal de détection positif, tandis que les partitions ne contenant pas de séquence nucléotidique cible ne sont pas amplifiées et ne produisent pas de signal de détection ;
comprenant en outre les étapes suivantes :
a) fourniture d'un ensemble de données comprenant une pluralité de valeurs d'intensité provenant d'une pluralité d'échantillons de mesure ; dans lequel ladite pluralité d'échantillons de mesure est dérivée d'un seul échantillon d'essai biologique comprenant des acides nucléiques ;
b) fourniture d'une valeur de discrimination prédéfinie destinée à séparer les valeurs d'intensité en deux catégories de données différentes ;
c) séparation de la pluralité de valeurs d'intensité dans l'une ou l'autre des deux catégories de données différentes par détermination que les valeurs individuelles des valeurs d'intensité sont au-dessus ou au-dessous de la valeur de discrimination prédéfinie ;
d) détermination de la fonction de probabilité cumulative pour toutes les valeurs dans la catégorie de données au-dessus de la valeur de discrimination, Groupe A, et détermination de la fonction de probabilité cumulative pour toutes les valeurs dans la catégorie de données au-dessous de la valeur de discrimination, Groupe B ;
e) obtention d'une nouvelle valeur de discrimination telle qu'un rapport entre les deux fonctions de probabilité cumulative correspond à un facteur d'erreur prédéfini ;
f) itération des étapes c) à e) ;
- moyennant quoi la nouvelle valeur de discrimination obtenue à l'étape e) après une itération remplace la valeur de discrimination de l'itération précédente ; et
- moyennant quoi l'itération est réalisée jusqu'à ce que les compositions des catégories de données, positives pour le Groupe A et négatives pour le Groupe B, restent constantes ou sur un nombre prédéterminé d'itérations ; et
g) fourniture automatique de la nouvelle valeur de discrimination obtenue à l'étape f) de la dernière itération comme seuil pour l'affectation de valeurs d'intensité dérivées d'un dosage par PCR numérique à partir de l'échantillon biologique comprenant des acides nucléiques dans l'une ou l'autre catégorie de données, positives pour le Groupe A et négatives pour le Groupe B.

2. Procédé de la revendication 1, dans lequel lesdites valeurs d'intensité sont des valeurs d'intensité de fluorescence, chimioluminescence, rayonnement ou changements colorimétriques.

3. Procédé de l'une quelconque des revendications 1 à 2, dans lequel ladite valeur de discrimination prédéfinie destinée à séparer les valeurs d'intensité en deux catégories de données différentes est la médiane, la moyenne arithmétique ou n'importe quel percentile entre le 15^{e} et le 85^{e} percentile des valeurs d'intensité.

4. Procédé de l'une quelconque des revendications 1 à 3, dans lequel l'étape e) comprend :
i. la détermination du point d'intersection entre les fonctions de probabilité cumulative de l'étape d) ;
ii. le calcul de la probabilité pour que ledit point d'intersection dérive deux nouvelles fonctions de probabilité cumulative ;
iii) la multiplication ou division desdites nouvelles fonctions de probabilité cumulative par la racine carrée du facteur d'erreur prédéfini et le calcul de deux points d'approximation au moyen de fonctions de probabilité cumulative inverses ;
v) l'interpolation du rapport de fonctions de densité de probabilité des points d'approximation pour obtenir ainsi une nouvelle valeur de discrimination.

5. Procédé de l'une quelconque des revendications 1 à 4, dans lequel le facteur d'erreur est inférieur ou égal à 100, inférieur ou égal à 80, inférieur ou égal à 60, inférieur ou égal à 50, inférieur ou égal à 40, inférieur ou égal à 30, inférieur ou égal à 20 ou inférieur ou égal à 10.

6. Procédé de l'une quelconque des revendications 1 à 5, dans lequel ledit nombre prédéterminé d'itérations est n'importe quel nombre entre 5 et 20, entre 5 et 15, entre 5 et 12 ou entre 5 et 10.

7. Procédé de l'une quelconque des revendications 1 à 6, comprenant en outre l'étape suivante :
h) obtention d'un nombre constant de points de données dans le Groupe A et le Groupe B.

8. Procédé de l'une quelconque des revendications 1 à 7, dans lequel le facteur d'erreur est inférieur ou égal à 10.

9. Procédé de l'une quelconque des revendications 1 à 8, dans lequel le facteur d'erreur est une espérance prédéfinie concernant la présence de faux positifs et faux négatifs.

10. Procédé de l'une quelconque des revendications 1 à 9, comprenant en outre l'étape suivante : i) affectation des données de mesure dans l'une ou l'autre des deux catégories de données différentes.

11. Procédé de l'une quelconque des revendications 1 à 10, dans lequel l'ensemble de données est un ensemble de données récupéré par réalisation d'une PCR numérique en gouttelettes.

12. Support lisible par ordinateur comprenant un code de programme informatique qui, lorsqu'il est exécuté sur un dispositif de traitement de données, réalise le procédé de l'une quelconque des revendications 1 à 11.

13. Dispositif servant à réaliser le procédé de l'une quelconque des revendications 1 à 11 comprenant :
a) une unité de stockage de données comprenant une pluralité de valeurs d'intensité provenant d'une pluralité d'échantillons de mesure ; et
b) une unité de traitement de données dans laquelle est intégré de façon tangible un code de programme informatique réalisant le procédé de l'une quelconque des revendications 1 à 11.

14. Dispositif de la revendication 13, ledit dispositif comprenant en outre une unité de mesure pouvant obtenir les valeurs d'intensité à partir des échantillons de mesure et, de préférence, une unité d'analyse pouvant réaliser un dosage analytique.
